Europäisches Patentamt

European Patent Office

Office européen des brevets

(11) Publication number: 0 327 169 A2

# EUROPEAN PATENT APPLICATION

(21) Application number: 89200178.5

(51) Int. Cl.⁴: A61K 47/00 , A61K 39/395

(22) Date of filing: 27.01.89

(30) Priority: 02.02.88 US 151499

(43) Date of publication of application:
09.08.89 Bulletin 89/32

(84) Designated Contracting States:
AT BE CH DE ES FR GB GR IT LI NL SE

(71) Applicant: AKZO N.V.
Velperweg 76
NL-6824 BM Arnhem(NL)

(72) Inventor: Ebert, Raymond Francis
19029 Raines Drive
Derwood, MD 20855(US)

(74) Representative: Hermans, Franciscus G.M. et al
Patent Department AKZO N.V. Pharma
Division P.O. Box 20
NL-5340 BH Oss(NL)

(54) Immunotoxins from barley toxin.

(57) A process for making a conjugated immunotoxin by the steps of reacting barley toxin with a linking agent to introduce into the barley toxin not more than about two linking groups per barley toxin molecule to produce a bioactive, conjugable barley toxin, and reacting the conjugable barley toxin with an activated antibody to produce a bioactive immunotoxin.

Fig. 3

RETICULOCYTE LYSATE ASSAY
Barley Toxin

Legend:
··▲·· BT-SH 4.4
─●─ BT-SH 1.4
─○─ BT

X-axis: Log Concentration (mol/1)
Y-axis: % Inhibition

EP 0 327 169 A2

# Immunotoxins from barley toxin

The invention provides a conjugated immunotoxin and a method of making a conjugated immunotoxin.

An immunotoxin is a molecule containing a toxic substance covalently linked (conjugated) to an antibody. Due to its ability to react specifically with a target antigen, the antibody serves as a delivery vehicle for the toxin. Conversely, it is a desirable characteristic of the toxin that it does not bind to the surface of target or non-target cells. Current techniques provide a wide range of suitable monoclonal antibodies that can be conjugated to toxins to produce immunotoxins for the treatment of cancers (see Bjorn et al., Cancer Research 45, 1214-1221, 1986).

There are a variety of ways to conjugate a toxin to an antibody. Heterobifunctional cross-linking agents such as N-succinimidyl-3(2-pyridyldithio)-propionate (SPDP), are often used to provide a bridge between the antibody and the toxin. As an example of how immunotoxins are constructed, SPDP is reacted with an antibody to form a product consisting of the antibody covalently attached to pyridyldithione via a peptide bond involving an -NH₂ group on the antibody. The antibody that has been "activated" in this manner is then combined with (e.g.) a toxin that contains -SH (sulfhydryl or thiol) group(s).

The sulfhydryl groups on the toxin displace thiopyridine to form a new disulfide bond between the activated antibody and the toxin. By substituting other crosslinking agents for SPDP, it is also possible to form thioether or amide bonds in linking groups between activated antibody and toxin [Pastan et al., Cell 47: 641-648, (1986); Ghose et al. Meth Enzymol. 93: 280-333, (1983); Gilliland, et al., Proc. Nat. Acad. Sci., USA Vol. 75 pg. 5326, (1978)].

If a toxin of interest does not have -SH groups, and the crosslinking method described above is desired, then it is necessary to introduce new -SH groups into the toxin. This is accomplished, for example, by making use of a thiolating agent such as 2-iminothiolane [Jue et al. Biochem. 17: 5399-5406 (1978); Lambert et al. Biochem. 17: 5406-5416 (1978)]. This thiolating agent forms a covalent bond with -NH₂ groups on the toxin. The thiolated toxin is then reacted with an activated antibody to produce an immunotoxin. Possible thiolating agents, including SPDP, have been reviewed in Cumber et al., Meth. Enzymol. 112, 207-225 (1985).

Many possibly suitable toxins for preparing immunotoxins have been identified in plants [Coleman and Roberts, Biochem. Biophys. Acta 696, 239-244 (1982); Stirpe and Barbieri, FEBS Letters 195, 1-8 (1986)]. However, the number of toxins that have been used successfully in producing immunotoxins is relatively small. These include diphtheria toxin, pseudomonas exotoxin A, ricin, abrin, pokeweed antiviral protein, saporin, and gelonin (Pastan et al., vide supra). There is a need to find a way to use additional toxins in producing immunotoxins.

We have now identified barley toxin as a toxin that may be used for the production of a new series of immunotoxins. Barley toxin is a protein of approximately 30,000 Daltons isolated from barley seeds (Coleman and Roberts, vide supra). This toxin is a member of a group of toxins known as ribosome-inactivating proteins (RIP). Immunotoxins made from such proteins have been used to remove T-lymphocytes from bone marrow heterografts, preventing the graft-versus-host reaction.

It is an object of the present invention to provide a new series of immunotoxins constructed from barley toxin, a toxin that is relatively inexpensive and readily available. It is also an object of this invention to provide a process for making such an immunotoxin. It is a feature of the invention that the immunotoxin produced retains the cytotoxicity of the toxin. These and other objects, features and advantages are accomplished by the present invention, which provides a process for making a conjugated immunotoxin by the steps of (I) introducing linking groups on about two amino groups on the barley toxin to produce a bioactive conjugable toxin, and (II) reacting the bioactive toxin with an activated antibody to produce an immunotoxin.

In the preferred embodiment no more than two amino groups on the barley toxin are coupled to a linking agent containing thiol or disulfide groups, which are available or may be rendered available for linking to the activated antibody.

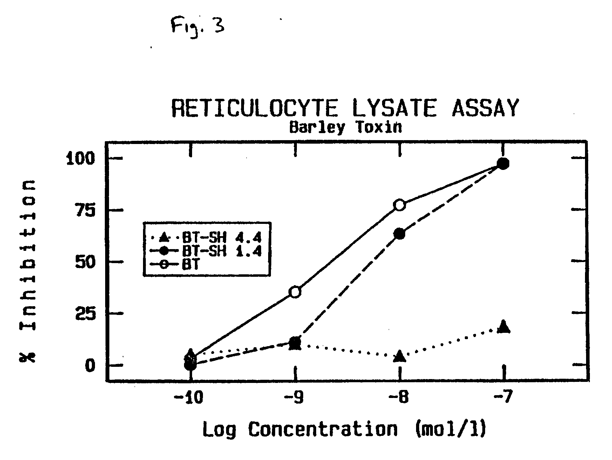

It is an advantage of the present invention that neither the affinity and specificity of the antibody, nor the cytotoxicity of the toxin is destroyed as a result of the method of conjugation. We have found that the solubility (and therefore the yield) of thiolated toxin is substantially decreased as the average number of -SH groups introduced into the toxin approaches or exceeds 5. We have also found (see Fig. 3) that barley toxin activity is lost if the average number of -SH groups introduced into the toxin exceeds 2. Therefore, it is preferred that the thiolating agent react with an average of no more than two -NH₂ groups per toxin molecule. Since the degree of thiolation is expressed as an average, it could vary by fractional amounts. It is preferred that this average be at least 0.5 and not more than 2.0.

Fig. 1 is a schematic diagram showing the use of SPDP in producing an activated monoclonal antibody, followed by conjugation to a toxin (designated "T") containing a free sulfhydryl group.

Fig. 2 is a schematic diagram showing the use of 2-iminothiolane to introduce a sulfhydryl group into barley toxin (designated "BT"), followed by conjugation to an activated monoclonal antibody.

Fig. 3 is a graph showing the relative toxicity of barley toxin and derivatives thiolated with 2-iminothiolane, in a cell-free protein synthesizing system using ribosomes from a reticulocyte lysate.

Fig. 4 is a graph showing the in vitro cytotoxicity against a colon tumor cell line (HT-29) of a barley toxin immunotoxin compared to unconjugated barley toxin and a thiolated derivative, using a tritiated leucine uptake assay.

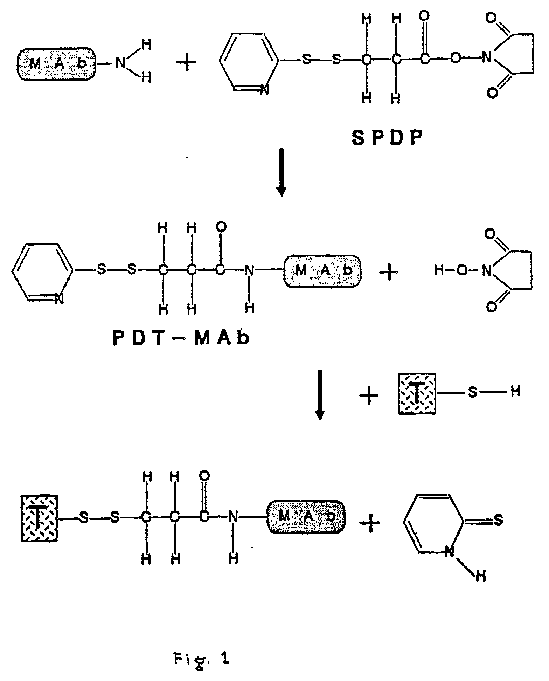

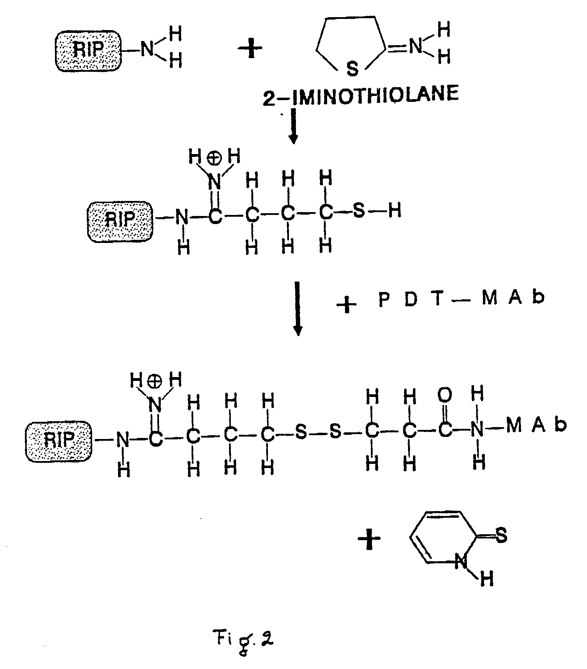

A series of useful immunotoxins based on barley toxin can be effectively produced in the following manner. With reference to Fig. 1, a monoclonal antibody (MAb) having a free -NH₂ group is activated with a heterobifunctional cross-linking agent, e.g. SPDP, to form an activated MAb, e.g. PDT-MAb. Then, as shown in Fig. 2, barley toxin (BT) is thiolated with 2-iminothiolane to introduce a free sulfhydryl group. The thiolated barley toxin is reacted with the activated MAb to form the disulfide-linked immunotoxin.

The following Example illustrates experimental conditions that were successfully used to introduce an average of about two thiol groups per barley toxin molecule. The parameters disclosed are for purposes of illustration and are not intended to limit the scope of the invention claimed.

## Example

### General:

Protein concentration was determined by the Lowry method (R.H. Lowry et al., J. Biol. Chem. 193, 265 (1951)) using bovine serum albumin as a standard. Protein thiolation was measured with Ellman's reagent (A.N. Glazer et al., in "Chemical Modification of Proteins", T.S. Work and E. Work, eds., Elsevier Biomedical Press, N.Y., 1975 p. 113) using a molar extinction coefficient (412 nm) of 13,600.

### Thiolation of barley toxin:

Introduction of not more than two thiol groups per barley toxin molecule may be achieved by controlling the pH, time, and temperature of the reaction and/or the concentration of the reactants. The following example illustrates reaction conditions that achieve this objective. Barley toxin [3-5 mg/ml in "thiolating buffer", 0.1 M potassium phosphate (pH 8.0) plus 0.001 M ethylenediaminetetraacetic acid (EDTA)] was incubated at 30 °C for 10 minutes with a 50-fold molar excess of 2-iminothiolane (prepared as a 0.4 M solution in thiolating buffer). The reaction mixture was dialyzed immediately at 4 °C against 400 volumes (three changes, 4-8 h. each) of 0.1 M potassium phosphate, (pH 7,0) plus 0.001 M EDTA.

### Activation of monoclonal antibody:

A murine anti-transferrin receptor monoclonal antibody was activated with N-succinimidyl-3-(2-pyridyldithio)-propionate (SPDP; Pharmacia Fine Chemicals, Piscataway, N.J.) according to the manufacturer's instructions, which are included herein by reference, using a SPDP:antibody ratio of 2 to 4.

### Coupling of barley toxin and monoclonal antibody:

Thiolated barley toxin and activated monoclonal antibody (toxin:antibody ratio of 3-5:1) were incubated for 4 h. at room temperature or overnight at 4 °C. Unconjugated barley toxin was separated from the immunotoxin by gel permeation chromatography, e.g., on a GF-250 XL Column (Dupont Co., Wilmington, DE) equilibrated with 0.05 M sodium phosphate (pH 7.5) plus 0.1 M NaCl.

### Results

The yield of thiolated barley toxin was usually in excess of 80% provided that, following incubation with 2-imino-thiolane, dialysis was carried out at pH 7.0. If dialysis was performed at pH 8.0 (the pH of the thiolation reaction) yields were usually less than 30%. The lower yields were likely the result of decreased solubility of the barley toxin due to incorporation of additional thiol groups, which proceeds faster at the higher pH.

The toxicity of the thiolated barley toxin resulting from the above procedure (average 1.4 sulfhydryl groups per toxin molecule) was compared, in Fig. 3 (dark circles) with non-thiolated barley toxin (open circles) and with barley toxin that had been thiolated to an average of 4.4 sulfhydryl groups per toxin molecule (dark triangles). The 1.4 sulfhydryl-group toxin was nearly as toxic as native barley toxin, while the 4.4 sulfhydryl-group toxin had lost virtually all toxicity. It was not possible to prepare barley toxin which had an average ratio of -SH:toxin greater than 5.0. Possibly, this was due to insolubility of the more highly substituted derivatives.

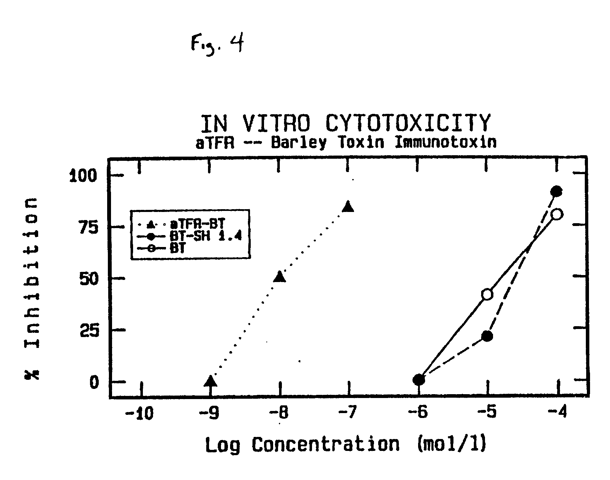

The in vitro cytotoxicity of the immunotoxin made by conjugating the 1.4 sulfhydryl-group toxin with a murine anti-transferrin receptor monoclonal antibody was compared in Fig. 4 (dark triangles) with the toxicity of barley toxin and 1.4 sulfhydryl-group thiolated barley toxin (open circles and dark circles, respectively). The immunotoxin was three orders of magnitude more effective at the 50% inhibition level than either nonconjugated toxin.

In another embodiment, the barley toxin may be coupled to an antibody via a thioether bond. This is accomplished by substituting a heterobifunctional cross-linking agent which contains a maleimidyl group (e.g. m-maleimidobenzoyl-N-hydroxysuccinimide ester) in place of the thiopyridine group on SPDP. Thus, the antibody is activated by covalent attachment of the crosslinking agent to antibody-NH$_2$ groups via the succinimidyl group. A thioether bond is then formed between the linker and the conjugable toxin via the maleimidobenzoyl group on the activated antibody, to produce an immunotoxin linked by a thioether bond.

It will be understood that the above description of the present invention is susceptible to various modifications, changes and adaptations, which are also intended to be comprehended within the meaning, range and equivalents of the appended claims.

## Claims

1. A process for making a conjugated immunotoxin, comprising the steps of:
reacting barley toxin with a linking agent to introduce into said barley toxin an average of no more than about two linking groups per barley toxin molecule to produce a conjugable and bioactive barley toxin; and
reacting said conjugable barley toxin with an activated antibody to produce a conjugated bioactive immunotoxin.

2. The process of claim 1, wherein the linking agent is a thiolating agent or heterobifunctional cross-linker, and the linking groups are sulfhydryl groups.

3. The process of claim 2, wherein the linking agent is 2-iminothiolane, and the immunotoxin is a disulfide-linked immunotoxin.

4. The process of claim 2, wherein the activated antibody contains a maleimidyl group, and the immunotoxin is a thioether-linked immunotoxin.

5. A process for making a conjugable and bioactive barley toxin, comprising the steps of:
reacting barley toxin with a linking agent to introduce into said barley toxin an average of no more than about two linking groups per barley toxin molecule, to produce a conjugable and bioactive barley toxin; and
recovering said conjugable and bioactive barley toxin.

6. The process of claim 5, wherein the linking agent is a thiolating agent and the linking groups are thiol groups.

7. The process of claim 5, wherein the linking agent is a heterobifunctional cross-linking agent and the linking groups are thiol or thioether groups.

8. An immunotoxin comprising:
a barley toxin molecule and
an antibody bonded covalently to the barley toxin molecule by an average of not more than about two linking groups.

9. The immunotoxin of claim 8 wherein the antibody is bonded covalently to the barley toxin molecule by an average of about one linking group.

10. The immunotoxin of claim 8 wherein the barley toxin molecule is bonded covalently linked to an average of about two linking groups.

Fig. 1

Fig. 2

EP 0 327 169 A2

Fig. 3

RETICULOCYTE LYSATE ASSAY
Barley Toxin

Fig. 4

IN VITRO CYTOTOXICITY
aTFR -- Barley Toxin Immunotoxin

% Inhibition

Legend:
▲ aTFR-BT
● BT-SH 1.4
○ BT

Log Concentration (mol/l)

EP 0 327 169 A2